# EUROPEAN PATENT SPECIFICATION

(11) **EP 3 061 439 B1**
(45) Date of publication and mention of the grant of the patent: **22.11.2017**
(21) Application number: 16000319.0
(22) Date of filing: 09.02.2016
(51) Int. Cl.: A61K 6/083, A61K 6/00

(54) **DENTAL GLASS IONOMER CEMENT COMPOSITION**
DENTALE GLASIONOMER-ZEMENTZUSAMMENSETZUNG
COMPOSITION DE CIMENT IONOMÈRE EN VERRE DENTAIRE

(30) Priority: 27.02.2015 JP 2015038255
(43) Date of publication of application: 31.08.2016
(73) Proprietor: GC Corporation, Bunkyo-ku Tokyo 113-0033 (JP)
(72) Inventor: Yoshimitsu, Ryosuke, Tokyo, 174-8585 (JP); Hokii, Yusuke, Tokyo, 174-8585 (JP); Yamamoto, Katsushi, Tokyo, 174-8585 (JP); Fukushima, Syouichi, Tokyo, 174-8585 (JP)
(74) Representative: Müller-Boré & Partner Patentanwälte PartG mbB

(56) References cited:
- WO-A1-02/053107
- GB-A- 2 353 042
- US-A- 5 084 491
- US-B1- 6 756 421

## Description

The present invention relates to a dental glass ionomer cement composition, more specifically, relates to a conventional dental glass ionomer cement composition that contains no polymerizable monomers.

Among dental cements, the dental glass ionomer cement is far superior in biological compatibility and adhesiveness to a tooth structure. Moreover, when it is set, or hardened, the set cement becomes translucent and superior in terms of esthetics, and is expected to have a gradual releasing effect of fluorine to strengthen the tooth structure. Therefore, the dental glass ionomer cement is used in a wide variety of applications in dentistry, including restoration.

This dental glass ionomer cement is a dental cement that includes fluoroaluminosilicate glass powder, polycarboxylic acid, and water as principal components. More specifically, the polyacrylic acid in water acts on the fluoroaluminosilicate glass powder to liberate metal ions (such as alkali metal ions, alkaline earth metal ions, and aluminum ions) in the glass, which are then coupled with carboxyl groups of the polyacrylic acid by ionic bonding to form crosslinking structures, and to be set (may be referred to as the "ionomer reaction", below).

Nowadays, resin-reinforced dental glass ionomer cements are also widely used that further contain polymerizable monomers in the conventional dental glass ionomer cement, to utilize polymerization reaction of the monomers in addition to the ionomer reaction. Compared with the conventional dental glass ionomer cement that contains no polymerizable monomers, this resin-reinforced dental glass ionomer cement has features such that the mechanical strength and adhesiveness to tooth structure are higher. On the other hand, since polymerizable monomers have irritating odor, and hence, may not be preferred, demand still exists for the conventional dental glass ionomer cement that contains no polymerizable monomers.

The conventional dental glass ionomer cement has a problem that the time required for the initial setting is comparatively longer because it mainly relies on the ionomer reaction. When using the dental glass ionomer cement in clinical practice, it is necessary to fill an application part having a cavity formed, with the dental glass ionomer cement, and then, to perform operations such as shape correction and surface polishing while spraying it with water. At this moment, if the cement contacts water before the initial setting has been sufficiently completed, metal ions may be eluted during the course of the setting reaction. This may result in a reduced strength of the set cement and/or a clouded surface of the set cement. To avoid these problems, a next clinical operation needs to wait. Therefore, in the field of the conventional dental glass ionomer cement, further shortening the initial setting time is still a problem to be tackled today.

Facing this problem, the applicant has developed in the past a setting method for the conventional dental glass ionomer cement containing no polymerizable monomers, that irradiates light on a mixture of fluoroaluminosilicate glass powder, polycarboxylic acid, and water, by which the initial setting time can be shortened (see, for example, Japanese Patent No. 4467672). Although a time-shortening effect has been obtained to a certain extent according to this method, it has been desired to shorten the initial setting time further.

Facing this problem, the applicant has further developed a conventional dental glass ionomer cement containing no polymerizable monomers, that contains a colorant to make the L* value represented in the L*a*b* colorimetric system be less than or equal to 60 under the standard illuminant D65 (see, for example, Japanese Patent No. 4498501). It has been confirmed that the initial setting time can be further shortened, by irradiating light on this composition.

However, to obtain the shortening effect of the initial setting time according to the Patent Document 2, the dental glass ionomer cement composition needs to have a colorant mixed to make its color show the L* value less than or equal to 60 represented in the L*a*b* colorimetric system, under the standard illuminant D65. Conversely, if the color has a high reflectance whose L* value is over 60, the shortening effect of the initial setting time cannot be obtained. This leads to demand for shortening the initial setting time of a dental glass ionomer cement composition that is closer to white color, desired from the esthetics point of view in recent years.

In view of the above, the problem to be solved by the present invention is to provide a dental glass ionomer cement composition that is a conventional dental glass ionomer cement containing no polymerizable monomers, and has no restriction on the L* value represented in the L*a*b* colorimetric system, under the standard illuminant D65, to obtain the shortening effect of the initial setting time, for example, for a color having a high reflectance whose L* value is over 60.

In order to solve the problem, the inventors have conducted intensive research, and consequently, found out that mixing a photopolymerization initiator, or a photopolymerization catalyst, into a conventional dental glass ionomer cement containing no polymerizable monomers, exhibits the shortening effect of the initial setting time, with no restriction on the L* value represented in the L*a*b* colorimetric system under the standard illuminant D65. Thus, the inventors completed the present invention.

That is to say, the present invention is a dental glass ionomer cement composition characterized by containing a photopolymerization initiator in an amount of 0.02% to 2% by weight with respect to a weight of the dental glass ionomer cement composition, and containing no polymerizable monomers.

According to the present invention, a dental glass ionomer cement composition is provided that is a conventional dental glass ionomer cement containing no polymerizable monomers, and has no restriction on the L* value represented in the L*a*b* colorimetric system under the standard illuminant D65, to obtain the shortening effect of the initial setting time.

### DESCRIPTION OF EMBODIMENTS

In the following, embodiments of the present invention will be described. Note that it will be apparent for one skilled in the art to make various modifications and replacements based on the embodiments within the scope of the present invention.

A dental glass ionomer cement composition according to an embodiment is characterized by containing a photopolymerization initiator in an amount of 0.02% to 2% by weigh, and containing no polymerizable monomers.

Here, the dental glass ionomer cement composition means a dental glass ionomer cement composition of the so-called "conventional type" that contains no polymerizable monomers, and contains fluoroaluminosilicate glass powder, polyacrylic acid, and water as principal components.

As fluoroaluminosilicate glass powder that can be used for the dental glass ionomer cement composition according to the embodiment, one may consider fluoroaluminosilicate glass powder that is generally used for dental glass ionomer cements. A preferable composition with respect to the total weight of the glass is, for example, 10% to 25% by weight of Al³⁺; 5% to 30% by weight of Si⁴⁺; 1% to 30% by weight of F⁻; 0% to 20% by weight of Sr²⁺; 0% to 20% by weight of Ca²⁺; and 0% to 10% by weight of alkali metal ions (such as Na⁺ and K⁺). The powder may be preferably prepared by mixing raw materials including the above, melting the mixture, and then, cooling and pulverizing the mixture into powder having the average grain diameter of 0.02 to 20 µm.

As a polycarboxylic acid that can be used for the dental glass ionomer cement composition according to the embodiment, a polycarboxylic acid generally used for dental glass ionomer cements can be used, which may be homopolymers and copolymers of α,β-unsaturated monocarboxylic acid or α,β-unsaturated dicarboxylic acid. Specific examples of α,β-unsaturated monocarboxylic acid or α,β-unsaturated dicarboxylic acid include acrylic acid, methacrylic acid, 2-chloroacrylic acid, 3-chloroacrylic acid, aconitic acid, mesaconic acid, maleic acid, itaconic acid, fumaric acid, glutaconic acid, and citraconic acid. The weight-average molecular weight of the polycarboxylic acid is preferably 5,000 to 40,000. The polycarboxylic acid is usually mixed into a liquid component, but may be mixed into a powder component.

The form of the dental glass ionomer cement composition according to the embodiment includes a powder-liquid type that has powder components and liquid components to be mixed and malaxated when they are used; a two-paste type that has a paste containing fluoroaluminosilicate glass powder, water, and a substance having a thickening effect, which is to be mixed with a polycarboxylic acid solvent when they are used; and a single-paste type that is a single paste of the composition having micro-capsulation applied so as not to start the ionomer reaction. Any of these types is applicable.

The dental glass ionomer cement composition according to the present invention includes a photopolymerization initiator. By having a photopolymerization initiator mixed, the shortening effect of the initial setting time can be obtained without restricting the L* value represented in the L*a*b* colorimetric system under the standard illuminant D65.

Here, the L*a*b* colorimetric system will be described. The L*a*b* colorimetric system was standardized by the International Commission on Illumination (CIE) in 1976, and is adopted in Japan as a Japanese Industrial Standard (JIS), namely, JIS Z8729 "Specification of Color of Materials according to the L*a*b* colorimetric system and the L*u*v* colorimetric system". Note that a light source specifically used for the dental glass ionomer cement composition according to the present invention includes the standard illuminant D65 that is specified in JIS Z8716 as "Standard illuminant used for comparison of surface colors, Fluorescent lamp D65 - form and performance". In the L*a*b* colorimetric system, the lightness is represented by L* having a numerical value of 0 to 100 where L*=0 represents pure black, and L*=100 represents pure white. Also, a* and b* represent the hue and the chroma represented by values of -60 to 60, respectively. These a* and b* represent directions of the color where a* represents a red direction,-a* represents a green direction, b* represents a yellow direction, and -b* represents a blue direction. A greater absolute value of a numerical value represents a brighter color, and a lesser represents a duller color.

Examples of the photopolymerization initiator include: camphorquinone; 2,2-dimethoxy-1,2-diphenylethane-1-on; 1-hydroxy-cyclohexylphenyl-ketone 2-hydroxy-2-methyl-1-phenyl-propane-1-on; 1-[4-(2-hydroxyethoxy)-phenyl]-2-hydroxy-2-methyl-1-propane-1-on; 2-hydroxy-1-{4-[4-(2-hydroxy-2-methyl-propionyl)-benzil] phenyl}-2-methylpropane-1-on; phenyl glyoxylic acid methyl ester; 2-methyl-1-(4-methylthiophenyl)-2-morpholinopropane-1-on; 2-benzil-2-dimethylamino-1-(4-morpholinophenyl)-butanone-1; 2-(dimethylamino)-2-[(4-methylphenyl) methyl]-1-[4-(4-morpholinyl) phenyl]-1-butanone; 2,4,6-trimethylbenzoyl-diphenyl-phosphine oxide; bis(2,4,6-trimethylbenzoyl)-phenylphosphine oxide; bis (η5-2,4-cyclopentadiene-1-yl)-bis(2,6-difluoro-3-(1H-pyrrole-1-yl)-phenyl) titanium; 1.2-octanedion,1-[4-(phenylthio)-,2-(O-benzoyloxime)]; ethanone,1-[9-ethyl-6-(2-methylbenzoyl)-9H-carbazole-3-yl]-,1-(O-acetyloxime); thioxanthone; anthraquinone; and 2-ethylanthraquinone. Especially preferable ones among these are: camphorquinone; bis(2,4,6-trimethylbenzoyl)-phenylphosphine oxide; and 2,4,6-trimethylbenzoyl-diphenyl-phosphine oxide. Also, the absorption wavelength region of a photopolymerization initiator is preferably 320 to 780 nm because a generally-used dental light irradiator can be used. A further preferable absorption wavelength region of the photopolymerization initiator is 400 to 520 nm.

The amount of a photopolymerization initiator mixed into the dental glass ionomer cement composition may be 0.01% to 5% by weight. If it is less than 0.01% by weight, there is a tendency that the shortening effect of the initial setting time is not obtained; or if it is over 5% by weight, the amount of the glass component is relatively reduced, and there is a tendency that the strength is not obtained. The amount of the photopolymerization initiator according to the invention is 0.02% to 2% by weight.

The photopolymerization initiator may be contained in advance in any components among the fluoroaluminosilicate glass, the polycarboxylic acid, and water, and any other components constituting the dental glass ionomer cement composition in the form of powder, liquid, and paste.

The dental glass ionomer cement composition according to the embodiment contains no polymerizable monomers. Therefore, the dental glass ionomer cement composition according to the embodiment does not have the problem of irritating odor caused by polymerizable monomers contained in a resin-reinforced dental glass ionomer cement.

Further, if necessary, the dental glass ionomer cement composition according to the embodiment may have an ultraviolet light absorber, a plasticizer, an antioxidant, an antibacterial, a surfactant, a colorant, and the like added, that may be publicly known.

Next, use examples of the dental glass ionomer cement composition according to the embodiment will be described. First, an application part having a caries or the like removed by drilling or the like and having a cavity formed, is filled or applied with a malaxated dental glass ionomer cement. Next, the filled or applied dental glass ionomer cement is irradiated with light. Then, the finishing such as shape correction, surface polishing, etc., is performed while spraying it with water.

As a device used for irradiating light, an infrared-, visible- or ultraviolet-light irradiator widely used in the dentistry now can be used, which may use a light source capable of emitting light in a range of wavelengths of 320 to 3,000 nm. If the wavelength is less than 320 nm, it is harmful to a living organism. On the other hand, light in the wavelength region over the wavelength of 3000 nm tends to have a reduced effect in promoting the setting reaction of a dental glass ionomer cement. From the viewpoint of availability for dentists, a visible-light irradiator is preferable that is capable of irradiating light having the wavelength of 320 to 780 nm.

In the setting method of the dental glass ionomer cement according to the present embodiment, irradiating light in the range of wavelengths of 320 to 3,000 nm raises the temperature of the dental glass ionomer cement within an appropriate time to promote the setting reaction. Therefore, the irradiating is preferably executed by irradiance of at least 200 mw/cm².

### [Examples]

In the following, examples and comparative examples of the present invention will be described in further detail, but the present invention is not limited to these examples. Note that all of the used dental glass ionomer cement compositions are dental glass ionomer cement compositions of the so-called "conventional" types that contain no polymerizable monomers.

### <Preparation of fluoroaluminosilicate glass powder>

First, a batch was obtained by having 22 g of aluminum oxide, 23 g of silica, 12 g of calcium fluoride, 15 g of calcium phosphate, and 28 g of strontium fluoride, sufficiently mixed and stirred in a mortar. Then, the batch was put into a porcelain crucible, to raise the temperature to 1,200 °C in an electric furnace at the temperature rising speed of about 7 °C/min., and moored for three hours. After that, the melt was poured into water to form rapidly-cooled glass, and the glass was pulverized into fluoroaluminosilicate glass powder. The average grain diameter of this powder was 2.5 µm.

### <Preparation of dental glass ionomer cement composition>

Powder components and liquid components were prepared by a composition (unit is % by weight) illustrated in Table 1, and 0.40 g of the powder components, and 0.12 g of the liquid components were weighed to fill a capsule used for a dental capsule mixer (product name: Capsule Mixer CM-II, manufactured by GC Corp.).

**Table 1**

| (unit: % by weight) | | | Ex. 1 | Ex. 2 | Ex. 3 | Ex. 4 | Comp. Ex. 1 | Comp. Ex. 2 | Comp. Ex. 3 |
|---|---|---|---|---|---|---|---|---|---|
| Powder components | fluoroaluminosilicate glass powder | | 76.92 | 76.92 | 76.92 | 76.92 | 76.92 | 76.92 | 76.92 |
| | photopolymerization initiator | IRGACURE 819 | 1.51 | | | | | | |
| | colorants | Food blue No. 1 | | | | | 0.76 | | |
| | | Yellow colorant | | | | | | 0.06 | |
| Liquid components | polycarboxylic acid solution | 48%-solution | 21.57 | 23.03 | 23.03 | 22.99 | 22.32 | 23.02 | 23.08 |
| | photopolymerization initiator | camphorquinone | | 0.05 | | | | | |
| | | Lucirin TPO | | | 0.05 | 0.09 | | | |
| Evaluation result | | start time of water-spraying and polishing | 2 min | 2 min | 2 min | 2 min | 2 min | 2min 30s | 2min 30s |
| | | lightness (L*) | 79.5 | 77 | 77.3 | 77.7 | 23.2 | 76.4 | 76.8 |

The official names of the materials in the table are as follows:
IRGACURE 819 (made by BASF): bis(2,4,6-trimethylbenzoyl)-phenylphosphine oxide; Lucirin TPO (made by BASF): 2,4,6-trimethylbenzoyl-diphenyl-phosphine oxide;
Food blue No. 1 (made by DaiwaKasei Co.,Ltd.): Brilliant blue FCF; and
Yellow colorant (made by Ciba-Geigy Japan Ltd.): Chromophtal yellow.

### <Evaluation of start time of water-spraying and polishing>

Capsules prepared as described above were malaxated for 10 seconds, respectively, by using a dental capsule mixer (product name: Capsule Mixer CM-II, manufactured by GC Corp.). After the malaxation, by using a capsule applier (product name: Capsule Applier IV, manufactured by GC Corp.), an acrylic mold having the internal diameter of 5 mm and the height of 2 mm was filled with the composition, and applied with pressure on a transparent OHP film to be molded. Immediately after that, it was put into a constant-temperature, constant-humidity chamber set at 37 °C, and the relative humidity of 100%. When one minute had passed since the start of malaxation, light was irradiated for 30 seconds, by using a light irradiator (product name: G-LightPrima II, manufactured by GC Corp.). When a predetermined time had passed since the start of malaxation, the OHP film was taken out, and the composition was immersed into 37 °C-distilled water for one minute. After the immersion, it was taken out of the water, the water was removed from the sample surface, and a desiccant (product name: Fuji VARNISH, made by GC Corp.) was applied. The sample having the desiccant applied was again immersed into 37 °C-distilled water. After having been immersed for 24 hours, it was taken out of the water, the desiccant was removed, and the hardness of the surface was measured by a micro Vickers hardness tester (product name: Micro Vickers Hardness Tester HMV-G21, manufactured by Shimadzu Corp.). The predetermined time was changed to measure the hardness of the surface. When the hardness of the surface reached 90% of the hardness value separately measured for a sample never having the first one-minite immersion in the water applied, yet having been immersed for 24 hours in the constant-temperature, constant-humidity chamber set at 37 °C, and the relative humidity of 100%, the time passed since the start of malaxation was defined as the start time of water-spraying and polishing. A shorter start time of water-spraying and polishing makes it possible to spray the application part with water in a shorter time, and to make the initial setting time shorter. The results are illustrated in Table 1.

### <Evaluation of lightness>

An acrylic ring having the diameter of 15 mm and the thickness of 1 mm was filled with the dental glass ionomer cement composition having been malaxated by the method, and applied with pressure on a transparent OHP film to be molded. Immediately after that, it was put into the constant-temperature, constant-humidity chamber set at 37 °C and the relative humidity of 100%. After one hour, the OHP film and the acrylic ring were taken out, and the L* value of the sample surface represented in the L*a*b* colorimetric system (lightness) under the standard illuminant D65 was measured by using a spectrophotometer (product name: Spectrophotometer CM-3610d, manufactured by Konica Minolta, Inc.) under a dehydrated condition, with a white color background. A higher L* value is evaluated to have a higher lightness. The results are illustrated in Table 1.

The evaluation results show that a dental glass ionomer cement composition containing a colorant exhibits a low lightness (Comparative example 1), or does not acquire the shortening effect of the initial setting time (Comparative example 2). Also, when neither of a photopolymerization initiator nor a colorant is contained, the shortening effect of the initial setting time is not acquired (Comparative example 3). On the other hand, dental glass ionomer cement compositions containing a photopolymerization initiator (Examples 1 to 4) have the short start time of water-spraying and polishing, and the set cements exhibit high lightness.

## Claims

1. A dental glass ionomer cement composition, comprising:
a photopolymerization catalyst,
wherein the amount of the photopolymerization catalyst mixed into the dental glass ionomer cement composition is 0.02% to 2% by weight with respect to the weight of the dental glass ionomer cement composition; and
wherein the dental glass ionomer cement composition contains no polymerizable monomers.

## Patentansprüche

1. Dentale Glasionomer-Zementzusammensetzung, umfassend:
einen Photopolymerisationskatalysator,
wobei die Menge des Photopolymerisationskatalysators, gemischt in die dentale Glasionomer-Zementzusammensetzung, 0,02 Gew.-% bis 2 Gew.-% bezüglich des Gewichtes der dentalen Glasionomer-Zementzusammensetzung beträgt, und
wobei die dentale Glasionomer-Zementzusammensetzung keine polymerisierbaren Monomere enthält.

## Revendications

1. Composition de ciment dentaire à base de verre ionomère comportant un catalyseur de photopolymérisation, dans laquelle la quantité de catalyseur de photopolymérisation mélangée à la composition de ciment dentaire à base de verre ionomère est de 0,02 % à 2 % en poids par rapport au poids de la composition de ciment dentaire à base de verre ionomère ; et dans laquelle la composition de ciment dentaire à base de verre ionomère ne contient pas de monomères aptes à la polymérisation.
